# EUROPEAN PATENT APPLICATION

(11) **EP 2 101 178 A1**
(43) Date of publication of application: **16.09.2009**
(21) Application number: 08152651.9
(22) Date of filing: 12.03.2008
(51) Int. Cl.: G01N 33/68

(54) **Use of Procalcitonin (PCT) in prognosis following acute coronary syndromes**

(71) Applicant: BRAHMS Aktiengesellschaft, 16761 Hennigsdorf (DE)
(72) Inventor: Bergmann, Andreas, 12351 Berlin (DE); Struck, Joachim, 13465 Berlin (DE); Ng, Leong Loke, Leicester, LE2 3NB (GB)
(74) Representative: Vossius & Partner

(57) **Abstract**

Subject of the present invention are assays and *in vitro* methods for determining a prognosis for a patient having an acute coronary syndrome, whereby the level of procalcitonin or fragments thereof is measured in a sample obtained from said patient. Said level of procalcitonin or fragments thereof may then be correlated to a predisposition to an adverse outcome of said acute coronary syndrome.

## Description

Subject of the present invention are assays and *in vitro* methods for determining a prognosis for a patient having an acute coronary syndrome, whereby the level of procalcitonin or fragments thereof is measured in a sample obtained from said patient. Said level of procalcitonin or fragments thereof may then be correlated to a predisposition to an adverse outcome of said acute coronary syndrome.

The term acute coronary syndromes (ACS) has been applied to a group of coronary disorders that result from ischemic insult to the heart. Patients with ACS form a heterogeneous group, with differences in pathophysiology, clinical presentation, and risk for adverse events. Such patients present to the physician with conditions that span a continuum that includes unstable angina, non-ST-elevation non-Q wave myocardial infarction (NST-MI), ST-elevation non-Q wave MI, and transmural (Q-wave) MI. ACS is believed to result largely from thrombus deposition and growth within one or more coronary arteries, resulting in a partial or complete occlusion of the artery, and frequently involves rupture of the plaque, resulting in an ischemic injury. ACS may also be precipitated by a coronary vasospasm or increased myocardial demand. For a review, see, e.g., Davies, Clin. Cardiol. 20 (Supp. I): I2-I7 (1997).

The seriousness of ACS is underlined by the morbidity and mortality that follow the ischemic insult. For example, it has been estimated that within four to six weeks of presentation with ACS, the risk of death or a subsequent MI is 8-14%, and the rate of death, MI, or refractory ischemia is 15-25% (Theroux and Fuster, Circulation 97: 1195-1206 (1998)). Given that the total number of deaths in the U.S. from acute MI is about 600,000, in the past the search for information that relates to the diagnosis, prognosis, and management of ACS has understandably been extensive. Several potential markers that may provide such information in certain patient populations have been identified, including circulating cardiac troponin levels (see, e.g., Antman et al., N. Eng. J. Med. 335: 1342-9 (1996); see also U.S. Pat. Nos. 6,147,688, 6,156,521, 5,947,124, and 5,795,725, ST-segment depression (see, e.g., Savonitto et al., JAMA 281: 707-13 (1999)), circulating creatine kinase levels (see, e.g., Alexander et al., Circulation (Suppl.) 1629 (1998)), circulating c-reactive protein levels (see, e.g., Morrow et al., J. Am. Coll. Cardiol. 31: 1460-5 (1998)), circulating levels of polypeptides originating from pre-proBNP (see, e.g., Wiviott SD, de Lemos JA, Morrow DA. Clin Chim Acta. 2004 Aug 16;346(2):119-28).

Procalcitonin (PCT) has become a well-established biomarker for sepsis diagnosis: PCT reflects the severity of bacterial infection and is in particular used to monitor progression of infection into sepsis, severe sepsis, or septic shock. It is possible to use PCT to measure the activity of the systemic inflammatory response, to control success of therapy, and to estimate prognosis (Assicot M et al.: High serum procalcitonin concentrations in patients with sepsis and infection. Lancet 1993, 341:515-8; Clec'h C et al.: Diagnostic and prognostic value of procalcitonin in patients with septic shock. Crit Care Med 2004;32:1166-9; Lee YJ et al.: Predictive comparisons of procalcitonin (PCT) level, arterial ketone body ratio (AKBR), APACHE III score and multiple organ dysfunction score (MODS) in systemic inflammatory response syndrome (SIRS), Yonsei Med J 2004, 45, 29-37; Meisner M.: Biomarkers of sepsis: clinically useful? Curr Opin Crit Care 2005, 11, 473-480; Wunder C et al.: Are IL-6, IL-10 and PCT plasma concentrations reliable for outcome prediction in severe sepsis? A comparison with APACHE III and SAPS II. Inflamm Res 2004, 53, 158-163). The increase of PCT levels in patients with sepsis correlates with mortality (Oberhoffer M et al.: Outcome prediction by traditional and new markers of inflammation in patients with sepsis. Clin Chem Lab Med 1999;37:363-368).

An increasing number of studies discusses the potential role of PCT in other infectious diseases like pneumonia, bacterial meningitis and malaria (Bugden SA, Coles C, Mills GD. The potential role of procalcitonin in the emergency department management of febrile young adults during a sustained meningococcal epidemic. Emerg Med Australas 2004, 16, 114-119; Chiwakata CB et al.: Procalcitonin as a parameter of disease severity and risk of mortality in patients with Plasmodium falciparum malaria. J Infect Dis 2001, 183, 1161-1164; Schwarz S et al.: Serum procalcitonin levels in bacterial and abacterial meningitis, Crit Care Med 2000, 28, 1828-1832).

In vitro-studies showed that PCT plays an important role during monocyte adhesion and migration and further has an effect on inducible nitric oxide synthase (iNOS) gene expression (Linscheid P et al.: Expression and secretion of procalcitonin and calcitonin gene-related peptide by adherent monocytes and by macrophage-activated adipocytes, Crit Care Med 2004, 32, 1715-1721; Wiedermann FJ et al.: Migration of human monocytes in response to procalcitonin, Crit Care Med, 2002, 30, 1112-1117; Hoffmann G et al.: Procalcitonin amplifies inducible nitric oxide synthase gene expression and nitric oxide production in vascular smooth muscle cells, Crit Care Med, 2002, 30, 2091-2095.).

The association between PCT levels and low-grade inflammation of the arterial wall in atherosclerosis has only recently been analyzed. Patent application EP 07015271.5 discloses the use of PCT in the risk stratification of patients suffering from stable coronary artery disease (CAD). However, it is unknown so far, whether PCT has prognostic power in acute coronary syndromes (ACS).

The use of B-type natriuretic peptide (BNP) and NT-proBNP in the prognosis of patients with acute coronary syndromes has been demonstrated in various studies (Mazzone M, Forte P, Portale G, Mancini F, Ursella S, La Sala M, Testa A, Covino M, Pignataro G, Gentiloni Silveri N.Brain natriuretic peptide and acute coronary syndrome.Minerva Med. 2005 Feb;96(1):11-8. Arakawa N, Nakamura M, Aoki H, Hiramori K. Plasma brain natriuretic peptide concentrations predict survival after acute myocardial infarction. J Am Coll Cardiol. 1996;27:1656-1661. de Lemos JA, Morrow DA, Bentley JH, et al. The prognostic value of B-type natriuretic peptide in patients with acute coronary syndromes. N Engl J Med. 2001;345:1014-1021. Omland T, Persson A, Ng L, et al. N-terminal pro-B-type natriuretic peptide and long-term mortality in acute coronary syndromes. Circulation. 2002;106:2913-2918.)

B-type natriuretic peptide (BNP or BNP-32) is a 32-amino acid neurohormone that is synthesized in ventricular myocardium and released into the circulation in response to ventricular dilation and pressure overload. The functions of BNP, like for atrial natriuretic peptide, include natriuresis, vasodilation, inhibition of the renin-angiotensin-aldosterone axis, and inhibition of sympathetic nerve activity. The precursor of BNP is synthesized as a 108-amino acid molecule, referred to as pre-proBNP, which is proteolytically processed into a 76-amino acid N-terminal peptide (amino acids 1-76), referred to as NT-proBNP and the 32-amino acid mature hormone, referred to as BNP or BNP-32 (amino acids 77-108). It has been suggested that each of these species, NT-proBNP, BNP-32, and pre-proBNP, can circulate in human plasma (see, e.g., Tateyama et al., Biochem. Res. Commun. 185: 760-767 (1992); Hunt et al., Biochem. Biophys. Res. Commun. 214: 1175-1183 (1995)). Pre-proBNP and NT-proBNP, as well as peptides which are derived from BNP, pre-proBNP and NT-proBNP, which are present in the blood as a result of proteolysis of BNP, NT-proBNP and pre-proBNP, are collectively described herein as polypeptides originating from pre-proBNP.

There is need for highly reliable markers and methods for prognosis for a patient having an acute coronary syndrome.

### BRIEF DESCRIPTION OF THE DRAWINGS

Figure 1: Log plasma PCT levels on discharge. Outcomes: 0 = event free survivor, 1 = patient revascularised (coronary artery bypass or percutaneous intervention), , 2 = patient readmitted with heart failure, 3 = patient died. Log PCT levels are significantly different (P < 0.001) by ANOVA, and log PCT levels are significantly higher in groups 2 and 3 compared to 0 (P < 0.001, with Bonferonni correction for multiple comparisons).
Figure 2: Kaplan Meier curves showing event free survival (for the endpoint death and heart failure), of patients stratified by below or above PCT median values. The median PCT values was 0.045 µg/L.

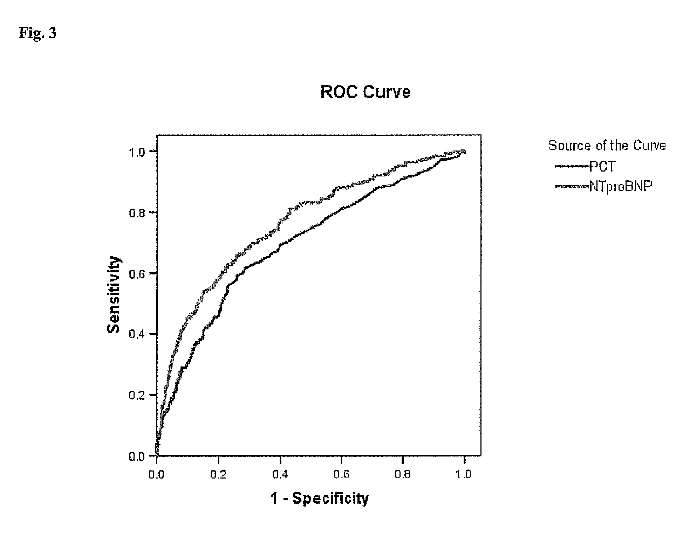

Figure 3: Receiver operating characteristic curves showing the prediction of the primary endpoint of Death or Heart failure using PCT (ROC AUC 0.69) and NTproBNP (ROC AUC 0.76).
Figures 4a/4b: Kaplan Meier curves showing event free survival (for the endpoint death and heart failure), of patients stratified by below (Figure 4a) or above (Figure 4b) PCT median values, and below or above NTproBNP median values. The median PCT value was 0.045 µg/L and the median NTproBNP value was 914 pmol/L.
Figures 5a/5b: Kaplan Meier curves showing event free survival (for the endpoint death), of patients stratified by below (Figure 5a) or above (Figure 5b) PCT median values, and below or above NTproBNP median values. The median PCT value was 0.045 µg/L and the median NTproBNP value was 914 pmol/L.

### DETAILED DESCRIPTION OF THE INVENTION

Subject of the present invention is an *in vitro* method for prognosis for a patient having an acute coronary syndrome, the method comprising: determining the level of procalcitonin or fragments thereof of at least 12 amino acids in length, preferably more than 50 aminoacids in length, more preferably more than 110 aminoacids in length in a sample obtained from said patient; and correlating said level of procalcitonin or fragments thereof to a predisposition to an adverse outcome of said acute coronary syndrome.

Acute coronary syndrome is an umbrella term used to cover any group of clinical symptoms compatible with acute myocardial ischemia. Acute myocardial ischemia is chest pain due to insufficient blood supply to the heart muscle that results from coronary artery disease (also called coronary heart disease).
An acute coronary syndrome (ACS) is a set of signs and symptoms, usually a combination of chest pain and other features, interpreted as being the result of abruptly decreased blood flow to the heart (cardiac ischemia); the most common cause for this is the disruption of atherosclerotic plaque in an epicardial coronary artery. The subtypes of acute coronary syndrome include unstable angina (UA, not associated with heart muscle damage), and two forms of myocardial infarction (heart attack), in which heart muscle is damaged. These types are named according to the appearance of the electrocardiogram (ECG/EKG) as *non-ST segment elevation myocardial infarction* (NSTEMI) and *ST segment elevation myocardial infarction* (STEMI).
ACS must be distinguished from coronary artery disease (also called coronary heart disease), which is atherosclerosis of the coronary arteries, a condition, where the arteries become clogged and narrowed, restricting blood flow to the heart, but where - as opoosed to ACS - the blood supply to the heart muscle is not blocked. Coronary artery disease may cause stable angina.

ACS should be distinguished from stable angina, which develops during exertion and resolves at rest, In contrast with stable angina, unstable angina occurs suddenly, often at rest or with minimal exertion, or at lesser degrees of exertion than the individual's previous angina ("crescendo angina"). New onset angina is also considered unstable angina, since it suggests a new problem in a coronary artery.

The term "acute coronary syndrome" encompasses a range of thrombotic coronary artery diseases, including unstable angina and both ST-segment elevation and non-ST-segment elevation myocardial infarction. Diagnosis requires an electrocardiogram and a careful review for signs and symptoms of cardiac ischemia.

In a preferred embodiment said adverse outcome is selected from the group consisting of death, heart failure and myocardial infarction.

In another preferred embodiment said correlating step comprises comparing said level of procalcitonin or fragments thereof to a threshold level, whereby, when said level of procalcitonin or fragments thereof exceeds said threshold level, said patient is predisposed to said adverse outcome.

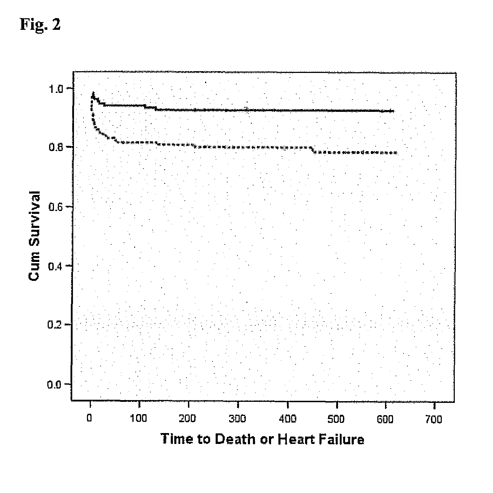

In a more preferred embodiment said threshold level is at about 0.045 (+/- 0.01) µg/L. Definition of this threshold value comes from the clinical study of the present document, which shows that at the median PCT concentration of the investigated patient population (0.045 µg/L), a strong discrimination of patients with adverse outcome from those without adverse outcome can be achieved (see Kaplan-Meier-Plot, Fig. 2)

The above-mentioned threshold level and the levels of markers are determined as described exemplary for PCT and NT-proBNP herein below in the examples. Hereby, generally the level of procalcitonin or alone or in combination with further prognostic markers may be assessed in the same manner.

In another preferred embodiment said sample is selected from the group comprising a blood sample, a serum sample, and a plasma sample.

In a further embodiment, the *in vitro* method for prognosis for a patient having an acute coronary syndrome further comprises mathematically combining said level of procalcitonin or fragments thereof with the level of one or more additional prognostic markers, whereby the combination of said level of procalcitonin or fragments thereof with said level of additional prognostic marker(s) increases the predictive value of said level of procalcitonin or fragments thereof or the level of said related marker for said adverse outcome. The mathematical combination can be for instance an algorithm categorizing patients according to whether their level of procalcitonin is above or below a certain threshold value and whether their level of marker X (and Y, Z...) is above or below a certain threshold value.

In a further preferred embodiment one of said additional prognostic marker(s) is pre-proBNP or fragments thereof (these can be proBNP or derivatives thereof, i.e. BNP or NT-proBNP, as described above) in a sample obtained from said patient.

Further markers which may be used as additional prognostic marker(s) may be selected from the group comprising troponin, myeloperoxidase, CRP, neopterin, GDF-15, ST2, cystatin-C, as well as the following peptides in form of their mature peptides, precursors, pro-hormones and associated prohormone fragments: atrial natriuretic peptide, adrenomedullin, endothelins, vasopressin.

In another more preferred embodiment said fragment of pre-proBNP is NT pro-BNP.

In another more preferred embodiment said fragment of pre-proBNP is BNP.

A further more preferred embodiment is the method according to the invention, further comprising determining the level of one or more additional prognostic markers in a sample obtained from said patient, and combining both said level of procalcitonin or fragments thereof and said level of one or more additional prognostic markers to said predisposition to an adverse outcome, whereby the combination of said level of procalcitonin or fragments thereof with said level of one or more additional prognostic markers increases the predictive value of said level of procalcitonin or fragments thereof for said adverse outcome.

In a further more preferred embodiment the combination between said level of procalcitonin or fragments thereof and said level of one or more additional prognostic markers is conducted with a mathematical algorithm. The mathematical combination can be for instance an algorithm categorizing patients according to whether their level of procalcitonin is above or below a certain threshold value and whether their level of marker X (and Y, Z...) is above or below a certain threshold value.

Another subject of the invention is the use of an ultrasensitive procalcitonin assay having a lower limit of detection of < 0.045 (+/- 0.010) µg/L for determining in a patient a predisposition to an adverse outcome of an acute coronary syndrome.

In another preferred embodiment of the ultrasensitive procalcitonin assay the assay is a sandwich assay comprising two antibodies against different moieties of procalcitonin.

In another more preferred embodiment of the ultrasensitive procalcitonin assay one antibody is against the calcitonin moiety of procalcitonin, and the other antibody is a monoclonal antibody against the katacalcin moiety of procalcitonin.

### Examples

974 consecutive acute coronary syndrome (ACS) patients admitted to the Coronary Care Unit of the Leicester Royal Infirmary were included in the study. The study complied with the Declaration of Helsinki and was approved by the local ethics committee; written informed consent was obtained from patients. ACS was diagnosed if a patient had chest pain lasting >20 minutes, diagnostic serial electrocardiographic (ECG) changes consisting of new pathological Q waves or ST-segment and T-wave changes, and a plasma creatine kinase-MB elevation greater than twice normal or cardiac troponin I level >0.1 ng/ml (Alpert JS et al., J Am Coll Cardiol. 2000; 36: 959-969). ACS was sub-categorised into ST segment elevation myocardial infarction (STEMI) or non-ST segment myocardial infarction (NSTEMI). Exclusion criteria were known malignancy or surgery in the month prior to the study. The estimated GFR (eGFR) of these subjects was calculated by the simplified formula derived from the Modification of Diet in Renal Disease (MDRD) study, recently validated in patients with HF (Smilde TD et al. Circulation. 2006; 114 : 1572-80).

Blood samples were drawn at 3 to 5 days after the onset of chest pain for determination of the levels of plasma procalcitonin (PCT) and NT-proBNP. After 15 minutes bed rest, 20mL blood was collected into tubes containing EDTA and aprotinin. All plasma was stored at -70°C until assayed in a blinded fashion in a single batch.

Transthoracic echocardiography was performed in patients using a Sonos 5500 instrument (Philips Medical Systems, Reigate, UK). A 16-segment left ventricular wall motion index (LVWMI) based on the American Society of Echocardiography mode was derived by scoring each LV segment (1=normal, 2=hypokinesis, 3=akinesis and 4=dyskinesis (Paradoxical Motion), and dividing the total by the number of segments scored. Left ventricular ejection fraction (LVEF) was calculated using the biplane method of discs formula (Schiller NB et al. J Am Soc Echocardiogr. 1989; 2: 358-367). Impaired LV systolic function was defined as an EF <40% or a LVWMI >1.8.

The NT-proBNP assay was based on a non-competitive assay as previously published (Omland T et al. Circulation. 2002; 106: 2913-2918). Sheep antibodies were raised to the N-terminal of human NT-proBNP and monoclonal mouse antibodies were raised to the C- terminal. Samples or NT-proBNP standards were incubated in C-terminal IgG-coated wells with the biotinylated N-terminal antibody for 24 hours at 4°C. Detection was accomplished with methyl-acridinium ester (MAE)-labelled streptavidin on a MLX plate luminometer (Dynex Technologies Ltd., Worthing, UK). The lower limit of detection was 0.3 pmol/l. There was no cross reactivity with atrial natriuretic peptide, BNP, or C-type natriuretic peptide.

Procalcitonin (PCT) was measured as described (Morgenthaler NG et al.: Clin Chem, 2002 May, 48(5), 788-790). Sheep antibodies were raised against the calcitonin moiety of PCT, and a mouse monoclonal antibody was raised against the katacalcin moiety of PCT. Tubes were coated with the anti-katacalcin antibody. The anti-Calcitonin antibody was labelled with MACN Acridiniumester (InVent GmbH, Hennigsdorf, Germany) and served as tracer. Dilutions of recombinant PCT in normal horse serum served as standards. 100 µl sample or standard were incubated in the coated tubes for 30 minutes, 200 µl tracer were added. After incubation for 2 h the tubes were washed 4 times with 1 ml of LIA wash solution (BRAHMS AG), and bound. Chemiluminescence was measured using a LB952T luminometer (Berthold, Germany).

The level of both procalcitonin (PCT) and NTproBNP was assessed for the prediction of the primary endpoint (death or heart failure {HF}). Death was also investigated as an individual secondary endpoint. Hospitalization for HF was defined as a hospital readmission for which HF was the primary reason. Endpoints were obtained by reviewing the Office of National Statistics Registry and by contacting each patient. There was a minimum 6 month follow-up of all surviving patients.

Statistical analysis was performed on SPSS Version 14 (SPSS Inc, Chicago, Illinois). The continuous variables in the two independent groups were compared using the Mann Whitney U test. Spearman's correlations were performed. To test the independent predictive power for death or HF of peptide levels, survival analyses using Cox proportional hazard modelling and Kaplan Meier models were conducted. Levels of NT-proBNP and PCT were normalised by logarithmic transformation. Thus, hazard ratios refer to a tenfold rise in the levels of these markers. Cox models were always constructed using the same variables entered simultaneously (which included variables statistically significant in univariate analyses at P < 0.10).

To compare the accuracy of NT-proBNP and PCT as markers, receiver-operating characteristic (ROC) curves were generated and the area under the curves (AUC) was calculated. A two tailed P value of less than 0.05 was deemed to be statistically significant.

The demographic features of the patient population are shown in Table 1. Median length of follow-up was 660 days with a range of 0-2837 days. No patient was lost to follow-up and the minimum length of follow-up for survivors was 187 days (about 6 months).

During follow-up, 200 (20.5%) patients died and 82 (8.4%) were readmitted with heart failure. There were 779 STEMI patients, 68% of whom received thrombolytic therapy.

**Table 1: Characteristics of Patients in the Study. Values are medians [range] or numbers (percentage).**

| | AMI Patients |
|---|---|
| Number | 974 |
| Age (in years) | 66[24-95] |
| Male Gender | 715 |
| eGFR (ml/min/1.73m² surface area) | 68.7[14.9-243.9] |
| NTproBNP (pmol/L) | 914 [0.3-28886.8] |
| Procalcitonin (µg/L) | 0.045 [0.007-9.7] |
| Previous Medical History | |
| Angina Pectoris (%) | 249 (25.5) |
| Myocardial infarction (%) | 163 (16.7) |
| Hypertension (%) | 426 (43.7) |
| Diabetes Mellitus (%) | 213 (21.8) |
| Heart Failure (%) | 56 (5.7) |
| STEMI(%) | 779 (79.9) |

### PCT levels (Univariate analysis)

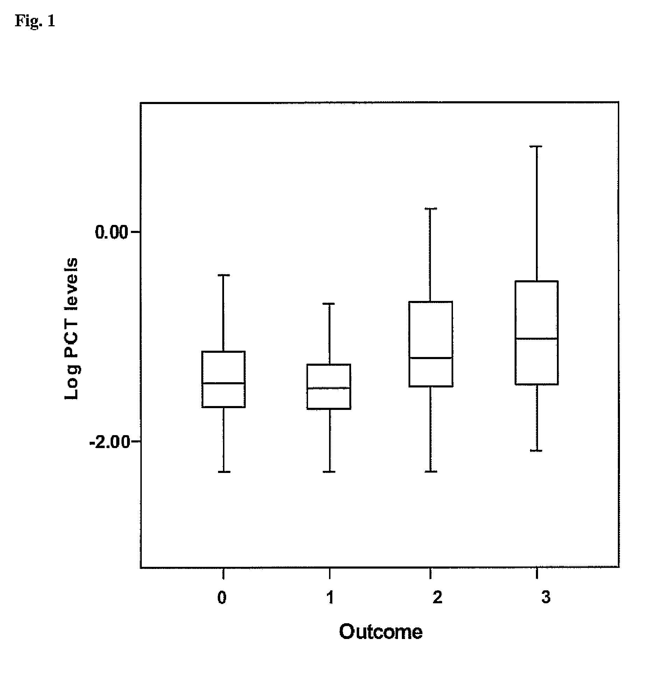

Plasma levels of PCT obtained predischarge (days 3-5) in 974 patients with ACS were elevated compared to the normal range (Table 2) (median PCT in normal individuals is 0.0127 µg/l (Morgenthaler NG et al.: Detection of procalcitonin (PCT) in healthy controls and patients with local infection by a sensitive ILMA. Clin Lab. 2002, 48, (5-6), 263-270). Male patients had lower levels of PCT compared to females (Table 2). Patients who had a previous history of acute myocardial infarction (AMI), hypertension, diabetes or heart failure (HF) had elevated PCT levels compared to those without such a past medical history. Levels of PCT were similar in those with anterior myocardial infarction compared to other sites of infarction, or STEMI compared to NSTEMI (Table 2). Those patients who had a Killip class higher than 1 (indicating presence of impaired left ventricular systolic function) had significantly elevated levels of PCT, and this was confirmed by the higher levels of PCT found in patients with echocardiographic evidence of systolic dysfunction (Table 2).
With regard to endpoints, PCT was raised in patients with death or HF compared to event-free survivors (Table 2). PCT was also raised in those patients with the individual secondary endpoints of death alone, or heart failure alone (Table 2, figure 1).
Plasma PCT was correlated with age, eGFR, Killip class (Table 3) and NT-proBNP (r = 0.36, P < 0.0005).

**Table 2: Univariate analysis of PCT and NT-proBNP in acute myocardial infarction (AMI) patients. Echocardiographic evidence for presence/absence ofHF was available for 639 patients.**

| (median [range]) | PCT (µg/L) | | P value | NTproBNP (pmol/L) | | P value |
|---|---|---|---|---|---|---|
| Males vs. Females | 0.035 vs. [0.003-6.23] | 0.045 [0.007-9.7] | <0.001 | 786 vs. [0.3-28886] | 1603 [5.7-24016] | <0.001 |

| **Previous Medical History** | | | | | | |
|---|---|---|---|---|---|---|
| AMI vs. none | 0.042 vs. [0.005-9.7] | 0.036 [0.003-6.2] | <0.034 | 1332 vs. [0.3-11259] | 842 [0.3-28886] | <0.002 |
| Hypertensi on vs. none | 0.039 vs. [0.004-6.23] | 0.035 [0.003-9.7] | <0.025 | 1103 vs. [0.3-28886] | 802 [0.3-24016] | <0.001 |
| Heart Failure vs. none | 0.051 vs. [0.011-6.23] | 0.036 [0.003-9.7] | <0.001 | 2817 vs. [9.4-12933] | 875 [0.3-28886] | <0.001 |
| Diabetes vs. none | 0.047 vs. [0.005-6.23] | 0.034 [0.003-9.7] | <0.001 | 1264 vs. [0.3-28886] | 829 [0.3-24016] | <0.001 |
| STEMI vs. NSTEMI | 0.036 vs. [0.005-9.7] | 0.039 [0.003-1.06] | NS | 1014 vs. [0.3-28886] | 662 [1.8-24016] | <0.001 |
| Anterior vs. other | 0.036 vs. [0.003-9.7] | 0.037 [0.004-3.63] | NS | 1009 vs. [0.3-24016] | 806 [0.3-28886] | NS |
| Thrombolysed vs. Not thrombolysed | 0.034 vs. [0.004-4.5] | 0.042 [0.003-9.7] | <0.001 | 883 vs. [0.3-15733] | 975 [0.3-28886] | NS |
| Killip class > 1 vs. Killip class 1 | 0.043 vs. [0.003-9.7] | 0.032 [0.004-2.47] | <0.001 | 1583 vs. [0.3-28886] | 630 [0.3-24016] | <0.001 |
| Echocardiograp hic evidence of HF vs. no HF | 0.051 vs. [0.005-9.7] | 0.032 [0.004-2.47] | <0.001 | 2286 vs. [0.3-14109] | 802 [0.3-28886] | <0.001 |

| **End Points** | | | | | | |
|---|---|---|---|---|---|---|
| Death or HF (n=248) vs. Event free survival | 0.071 vs. [0.003-9.7] | 0.032 [0.004-2.47] | <0.001 | 3595 vs. [2.4-28886] | 688 [0.3-24015] | <0.001 |
| Death (n=200) vs. Event free survival | 0.070 vs. [0.003-9.7] | 0.032 [0.004-2.47] | <0.001 | 3875 vs. [14.4-28886] | 688 [0.3-24015] | <0.001 |
| HF vs. Event free survival | 0.083 vs. [0.008-4.51] | 0.032 [0.004-2.47] | <0.001 | 3835 vs. [2.4-12933] | 688 [0.3-24015] | <0.001 |

| Spearman Correlations | | | | | | |
|---|---|---|---|---|---|---|
| Age | rₛ = 0.28 | | <0.001 | rₛ = 0.423 | | <0.001 |
| eGFR | rₛ = -0.329 | | <0.001 | rₛ = -0.426 | | <0.001 |
| Killip Class | rₛ = 0.231 | | <0.001 | rₛ = 0.323 | | <0.001 |

**Table 3. Cox regression analysis for death or HF post-AMI, showing univariate and multivariate analyses (using only significant predictive variables on univariate analysis).**

| | Univariate Analysis | | Multivariate Analysis | |
|---|---|---|---|---|
| | Hazard Ratio [95% CI] | P value | Hazard Ratio [95% CI] | P value |
| Age | 1.08 [1.07-1.09] | <0.001 | 1.04 [1.03-1.06] | <0.001 |
| Gender | 0.60 [0.46-0.77] | <0.001 | 1.05 [0.80-1.38] | NS |

| **Previous History of :-** | | | | |
|---|---|---|---|---|
| AMI | 2.51 [1.91-3.30] | <0.001 | 1.65 [1.24-2.19] | <0.001 |
| Heart Failure | 2.07 [1.32-3.24] | <0.002 | 0.98 [0.62-1.56] | NS |
| Hypertension | 1.59 [1.24-2.04] | <0.001 | 1.23 [0.95-1.60] | NS |
| Diabetes mellitus | 1.83 [1.40-2.39] | <0.001 | 1.21 [0.91-1.61] | NS |
| Anterior AMI | 1.05 [0.79-1.38] | NS | | |
| ST-elevation AMI | 0.82 [0.62-1.10] | NS | | |
| Thrombolytic use | 0.64 [0.50-0.82] | <0.001 | 0.80 [0.62-1.05] | NS |
| Killip class > 1 | 2.58 [1.96-3.38] | <0.001 | 1.26 [0.93-1.69] | NS |
| LogNTproBNP | 4.23 [3.36-5.34] | <0.001 | 2.47 [1.90-3.21] | <0.001 |
| Log PCT | 2.63 [2.18-3.18] | <0.001 | 1.29 [1.02-1.62] | <0.03 |
| eGFR | 0.96 [0.95-0.97] | <0.001 | 0.99 [0.98-1.00] | <0.04 |

### NT-proBNP levels (Univariate analysis)

Plasma NT-proBNP obtained in pre-discharge phase (days 3-5) was significantly higher in patients who died or were readmitted with HF compared to event-free survivors (Table 2). Significant differences in NT-proBNP levels were noted between males and females, those with a Killip class above 1 and in patients with a past medical history of heart failure, hypertension, myocardial infarction or diabetes (Table 2). Plasma NT-proBNP levels were also higher in STEMI vs. NSTEMI patients, but not in those with anterior site of AMI. Plasma NT-proBNP was correlated with age, eGFR and Killip class (Table 2).

### Prim Endpoints: PCT and NT-proBNP as predictors of death and heart failure

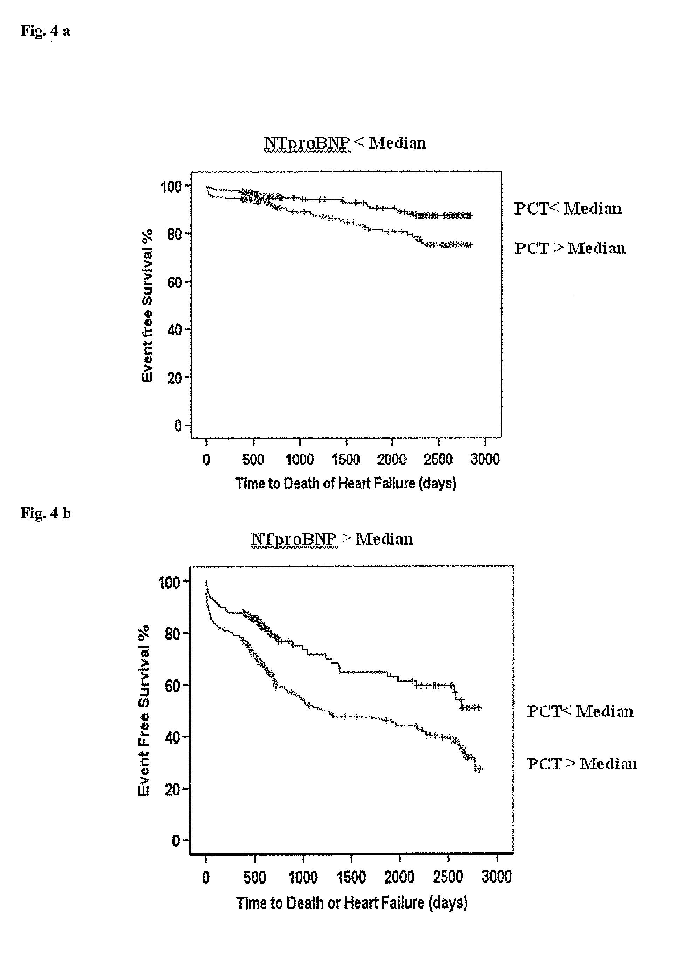

Univariate predictors of death or HF are reported in Table 3. The area under the receiver-operating-characteristic curve (AUC ROC) for PCT (0.69 [95% confidence interval CI: 0.65-0.73]) and NT-proBNP (0.76 [95% CI: 0.72-0.79]) were comparable.
Cox proportional hazards modelling using the univariate predictors that were significant (at up to P < 0.10) revealed NT-proBNP, PCT, age, eGFR and past history of AMI as independent and/or additional predictors of death or heart failure (Table 3). Neither Killip class, thrombolytic use, past history of heart failure, hypertension or diabetes were independent predictors.
The Kaplan-Meier survival curves plotting event free survival at below and above median PCT (0.045 µg/L) illustrate that those patients with above median PCT levels have a worse prognosis compared to those patients with a below median PCT (figure 2 (log rank, P<0.001)). The Kaplan-Meier survival curves plotting event free survival at below and above median PCT, at both below and above median values of NT-proBNP, illustrate that those patients with above median PCT levels have a worse prognosis compared to those patients with a below median PCT (figure 4, P < 0.009 (using log rank test) for patients who had below median NT-proBNP levels and P < 0.001 (using log rank test) for patients who had above median NT-proBNP levels).

### Secondary Endpoints: PCT and NT-proBNP as predictors of death

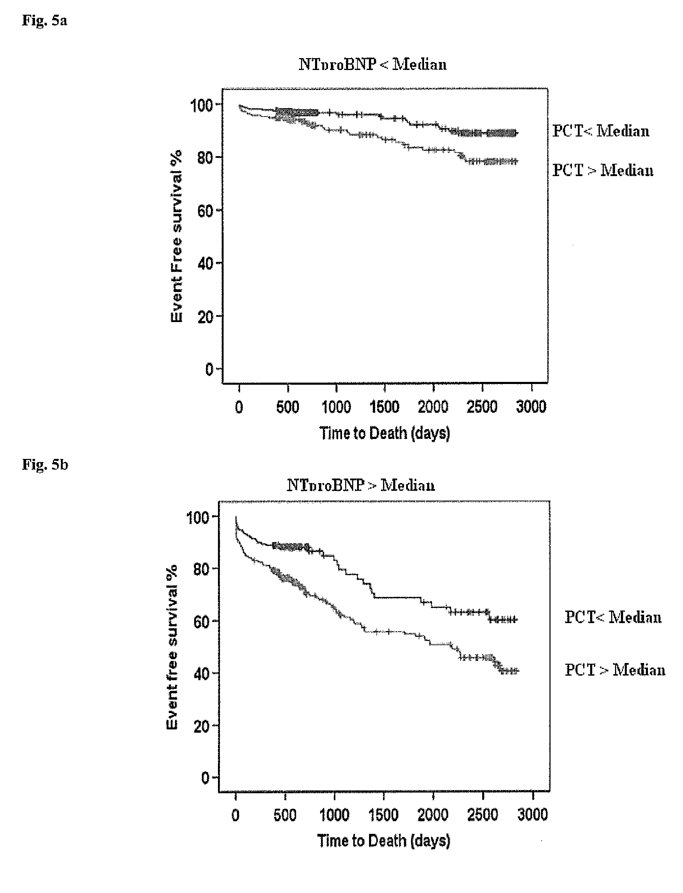

PCT and NT-proBNP were significantly higher in patients who died compared to event-free survivors (Table 2). Cox proportional hazards modelling suggested that the same variables (PCT, NT-proBNP, age and past history of AMI) were independent predictors of death. Kaplan-Meier analysis confirmed lower mortality in patients with PCT below the median in both the groups stratified by NT-proBNP median values (figure 5, P < 0.01 (using log rank test) for patients who had below median NT-proBNP levels, and P < 0.001 (using log rank test) for patients who had above median NT-proBNP levels).

**Table 4. Cox regression analysis for death post-AMI, showing univariate and multivariate analyses (using only significant predictive variables on univariate analysis).**

| | Univariate Analysis | | Multivariate Analysis | |
|---|---|---|---|---|
| | Hazard Ratio [95% CI] | P value | Hazard Ratio [95% CI] | P value |
| Age | 1.08 [1.07-1.10] | <0.001 | 1.05 [1.03-1.06] | <0.001 |
| Gender | 0.56 [0.42-0.4 | <0.001 | 0.96 [0.71-1.29] | NS |

| **Previous History of :-** | | | | |
|---|---|---|---|---|
| AMI | 2.51 [1.85-3.40] | <0.001 | 1.715 [1.25-2.35] | <0.001 |
| Heart Failure | 2.27 [1.41-3.66] | <0.001 | 1.08 [0.66-1.75] | NS |
| Hypertension | 1.50 [1.14-1.98] | <0.001 | 1.15 [0.86-1.53] | NS |
| Diabetes mellitus | 1.79 [1.33-2.41] | <0.001 | 1.17 [0.87-1.61] | NS |
| Anterior AMI | 1.05 [0.79-1.38] | NS | | |
| ST-elevation AMI | 0.82 [0.59-1.12] | NS | | |
| Thrombolytic use | 0.62 [0.47-0.82] | <0.001 | 0.79 [0.59-1.06] | NS |
| Killip class > 1 | 2.37 [1.7-3.20] | <0.001 | 1.09 [0.79-1.52] | NS |
| Log NTproBNP | 4.23 [3.36-5.34] | <0.001 | 2.52 [1.88-3.38] | <0.001 |
| Log PCT | 2.63 [2.18-3.18] | <0.001 | 1.30 [1.01-1.69] | <0.04 |
| eGFR | 0.96 [0.95-0.97] | <0.001 | 0.99 [0.98-1.00] | NS |

## Claims

1. An *in vitro* method for prognosis for a patient having an acute coronary syndrome, the method comprising: determining the level of procalcitonin or fragments thereof of at least 12 amino acids in length, in a sample obtained from said patient; and correlating said level of procalcitonin or fragments thereof to a predisposition to an adverse outcome of said acute coronary syndrome.

2. A method according to claim 1, wherein said adverse outcome is selected from the group consisting of death, heart failure and myocardial infarction.

3. A method according to claim 1 or 2, wherein said correlating step comprises comparing said level of procalcitonin or fragments thereof to a threshold level, whereby, when said level of procalcitonin or fragments thereof exceeds said threshold level, said patient is predisposed to said adverse outcome.

4. A method according to claim 3, wherein said threshold level is at about 0.045 (+/- 0.010) µg/L.

5. A method according to any of the preceding claims, wherein said sample is selected from the group comprising a blood sample, a serum sample, and a plasma sample.

6. A method according to any of the preceding claims, further comprising correlating said level of procalcitonin or fragments thereof with the level of one or more additional prognostic markers, whereby the combination of said level of procalcitonin or fragments thereof with said level of additional prognostic marker(s) increases the predictive value of said level of procalcitonin or fragments thereof or the level of said related marker for said adverse outcome.

7. A method according to claim 6, wherein one of said prognostic marker(s) is pre-proBNP or fragments thereof in a sample obtained from said patient.

8. A method according to claim 6 or 7, wherein said fragment of pre-proBNP is NT pro-BNP or BNP.

9. A method according to any one of claims 6 to 8, further comprising determining the level of one or more additional prognostic markers in a sample obtained from said patient, and correlating both said level of procalcitonin or fragments thereof and said level of one or more additional prognostic markers to said predisposition to an adverse outcome, whereby the combination of said level of procalcitonin or fragments thereof with said level of one or more additional prognostic markers increases the predictive value of said said level of procalcitonin or fragments thereof for said adverse outcome.

10. A method according to any of claims 6 to 9 wherein the additional prognostic marker is selected from a group comprising troponin, myeloperoxidase, CRP, neopterin, GDF-15, ST2, cystatin-C, as well as the following peptides in form of their mature peptides, precursors, pro-hormones and associated prohormone fragments: natriuretic peptides, adrenomedullin, endothelins, vasopressin.

11. A method according to any of claims 6 to 10, wherein the correlation between said level of procalcitonin or fragments thereof and said level of one or more additional prognostic markers is conducted with a mathematical algorithm.

12. Use of an ultrasensitive procalcitonin assay having a lower limit of detection of < 0.045 (+/- 0.010) µg/L for determining in a patient a predisposition to an adverse outcome of an acute coronary syndrome.

13. Use of an ultrasensitive procalcitonin assay according to claim 12, wherein the assay is a sandwich assay comprising two antibodies against different moieties of procalcitonin.

14. Use of an ultrasensitive procalcitonin assay according to claim 12 or 13, wherein one antibody is against the calcitonin moiety ofprocalcitonin, and the other antibody is a monoclonal antibody against the katacalcin moiety of procalcitonin.
